# EUROPEAN PATENT SPECIFICATION

(11) **EP 2 425 813 B1**
(45) Date of publication and mention of the grant of the patent: **18.04.2018**
(21) Application number: 10769483.8
(22) Date of filing: 23.04.2010
(51) Int. Cl.: A61K 8/891, A61K 8/34, A61K 8/892, A61K 8/894, A61K 8/898, A61Q 5/12

(54) **OIL-IN-WATER EMULSION-TYPE HAIR COSMETIC**
ÖL-IN-WASSER-EMULSIONS-HAARKOSMETIKPRÄPARAT
COSMÉTIQUE CAPILLAIRE DU TYPE ÉMULSION À PHASE CONTINUE AQUEUSE

(30) Priority: 27.04.2009 JP 2009107799
(43) Date of publication of application: 07.03.2012
(73) Proprietor: Shiseido Company, Ltd., Chuo-ku Tokyo 104-8010 (JP)
(72) Inventor: SHIMIZU, Hideki, Yokohama-shi Kanagawa 224-8558 (JP); FUJIYAMA, Taizo, Yokohama-shi Kanagawa 224-8558 (JP)
(74) Representative: Ter Meer Steinmeister & Partner
(86) International application number: PCT/JP2010/002951
(87) International publication number: WO 2010/125783

(56) References cited:
- EP-B1- 1 361 253
- JP-A- 9 165 317
- JP-A- 2000 247 842
- JP-A- 2002 020 221
- JP-A- 2002 348 216
- JP-A- 2004 051 584
- JP-A- 2005 220 076
- US-A1- 2008 292 574
- US-B1- 6 602 494

## Description

### TECHNICAL FIELD

The present invention relates to an oil-in-water emulsified hair cosmetic, and more specifically to an oil-in-water emulsified hair cosmetic that is used as a hair treatment and/or conditioner product and gives a good tactile sensation during use.

### BACKGROUND ART

Conventionally, silicone oil has been believed to be effective for improving damaged hair ends and tactile sensation after brushing and perm, and combinations of high polymer silicones and low boiling point oils have been widely used (Patent Document 1).

In recent years in particular, hair coloring has become a norm, which has made hair damage more severe, and therefore hair cosmetics for improving/repairing damaged hair are desired.

When hair is treated with hair color, the lipid layer on the hair surface is hydrolyzed due to the oxidant such as hydrogen peroxide contained in the hair color and cysteic acid is thus generated, which makes the hair hydrophilic; because of this, when a conventional oil-based cosmetic is used, there is a problem in that such hair surface treated with hair color results in poor absorption and spreadability and also oily-squeakiness occurs.

In terms of absorption into the hair, emulsified hair care agents, particularly the oil-in-water (O/W) emulsified hair cosmetics, have an advantage. However, the emulsified hair care agents usually end up having a turbid appearance. Therefore they are inferior in terms of the aesthetics of appearance and have the shortcoming of giving the impression of being sticky.

An emulsified cosmetic that avoids such shortcomings and is endowed with transparency has been developed (Patent Document 2). This emulsified cosmetic contains polyglyceryl fatty acid in the water phase and 30-70 wt% of the oil phase; however, due to the thickener and a high blend ratio of the oil component(s), it has the shortcoming of being sticky.

Patent Document 3 discloses a hair conditioning composition comprising polyalkylsiloxane mixture, aminosilicone and silicone copolymer emulsion.

### PRIOR ART DOCUMENTS

### PATENT DOCUMENTS

Patent Citation 1: Japanese Patent Laid-Open S63-183517 bulletin
Patent Citation 2: Japanese Patent Laid-Open 2005-220076 bulletin
Patent Citation 3: US 2008/0292574 A1

### DISCLOSURE OF INVENTION

### TECHNICAL PROBLEM

The object of the present invention is to provide an oil-in-water emulsified hair cosmetic that solves the aforementioned problems of conventional hair cosmetics, appears transparent or semi-transparent with superior aesthetics, manifests a high mending effect and richness as well as good tactile sensation during use.

### TECHNICAL SOLUTION

The inventors newly discovered an oil-in-water emulsified hair cosmetic that is an emulsified type, is absorbed well into the hair, can contain a large amount of oil components, has a rich sensation and a high mending effect as found in an oil-based hair care agent and yet appears transparent or semi-transparent. In the following, the unit for viscosity is expressed in the non-SI unit cs. It is noted that 1 cs equates to 1 mPa.s, whereby the latter is an SI unit for viscosity.

The present invention is a transparent or semi-transparent oil-in-water emulsified hair cosmetic characteristically comprising the following (a)-(d) wherein the blend ratio of the oil phase, including (a) and (b), is 50-80 wt% and the blend ratio (mass ratio) between (a) and (b) is (a) : (b) = 1 : 5 - 1 : 100.
(a) One, two or more silicones chosen from high polymer silicone, silicone with both ends hydroxy-modified, amino-modified silicone, ammonium-modified silicone, and polyalkylene glycol-modified silicone that are gum-like or have a viscosity of 1 million cs or higher; 0.1-10 wt%
(b) Dimethylpolysiloxane having a viscosity of 100 cs or less; 0.5-79 wt%
(c) Polyhydric alcohol; 1-25 wt%
(d) Hydrophilic surfactant; 1-10 wt%,
wherein transparent or semi-transparent means that the L value is 40 or higher when measured with a color-difference meter.

### ADVANTAGEOUS EFFECTS

The oil-in-water emulsified hair cosmetic of the present invention manifests good absorption and spreadability into the hair, doesn't cause dripping during use, and yet has the aesthetic appearance of transparent or semi-transparent gel despite the fact that it has a high mending effect and a rich sensation as found in oil-based hair care agents.

### BEST MODE FOR CARRYING OUT THE INVENTION

The embodiments of the present invention are described below.
((a) One, two or more silicones chosen from high polymer silicone, silicone with both ends hydroxy-modified, amino-modified silicone, ammonium-modified silicone, and polyalkylene glycol-modified silicone that are gum-like or have a viscosity of 1 million cs or higher)

Every ingredient in (a) used in the present invention is gum-like or has a viscosity of 1 million cs or higher.

The high polymer of the present invention is represented by the following general formula (1).

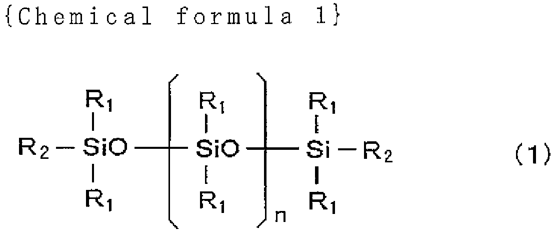

(R₁ denotes a methyl group or partially a phenyl group, and R₂ denotes a methyl or hydroxyl group. Also, n denotes an integer 3,000-20,000.)

For the high polymer silicone that is gum-like or has a viscosity of 1 million cs or higher, commercially available products can be used. Examples of the commercial products include BY11-040, BY11-003, FZ-3115, FZ-3132, BY11-007, BY11-014, and BY11-026 (from Dow Corning Toray Company Ltd.) and XF49-601, XF49-A3818, XF49-B7082, XF49-B7083, XF49-811, XF49-703, XF49-813, XF49-B1747, and XF49-B8324 (from Momentive Performance Materials, Inc.) and KF96H 1 million cs (from Shin-Etsu Chemical Co., Ltd.).

For the silicone with both ends hydroxy-modified of the present invention, commercial products can be used. Examples of the commercial products include XF49-C2499, XF49-C2520, XF49-C2497, and XF49-C2070 (from Momentive Performance Materials, Inc.) and 1501Fluid and 1503Fluid (from Dow Corning Toray Company Ltd.).

Amino-modified silicone and ammonium-modified silicone of the present invention are represented by the following formula (2):

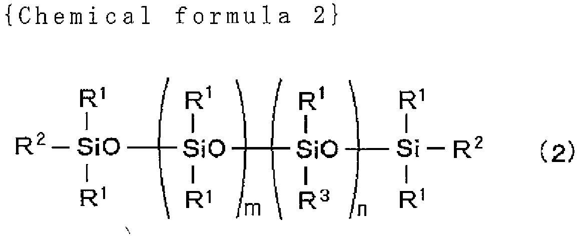

(In this formula, R¹ denotes a methyl group or partially a phenyl group, and is the same as R³ or denotes a methyl or hydroxyl group. R³ denotes a substituent having an amino group or an ammonium group represented by formula R⁴Z {R⁴ denotes a divalent alkylene group having 3 to 6 carbon atoms, Z denotes a monovalent group chosen from a group consisting of -NR⁵₂, -N⁺R⁵₃A⁻, -NR⁵(CH₂)ₐNR⁵₂, -NR⁵(CH₂)ₐN⁺R⁵₃A⁻ and -NR⁵(CH₂)ₐN(R⁵)C=O(R⁶) (R⁵ denotes hydrogen or an alkyl group having a hydrogen or 1-4 carbon atoms, R⁶ denotes an alkyl group having 1-4 carbon atoms, A denotes Cl, Br, or I, and a denotes an integer 2-6.)}, m and n each denotes a positive integer, m + n denotes an integer 3,000-20,000, and n/m is 1/500-1/10,000.)

The amino-modified silicone and ammonium-modified silicone of the present invention can be prepared with the same manner as general amino-modified silicone and ammonium-modified silicone. For example, they can be prepared by a polymerization/condensation reaction between γ - aminopropylmethoxysilane, cyclic methylpolysiloxane and hexamethyldisiloxane under the presence of an alkali catalyst.

The polyalkylene glycol-modified silicone of the present invention is represented by the following general formula (3).

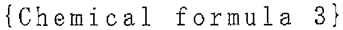

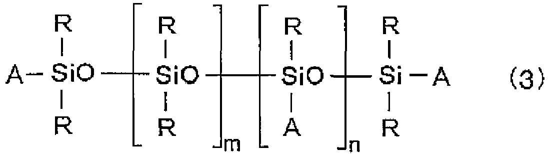

(In this formula, A denotes a group selected from a group consisting of a methyl group, phenyl group, and polyoxyalkylene group represented by a general formula -C₃H₆O(C₂H₄O)a(C₃H₆O)bR' (in this formula, R' is a group selected from a group consisting of hydrogen atom, acyl group, and alkyl group having 1-4 carbon atoms, and a and b each is an integer 5-50) (there is at least one polyoxyalkylene group in one molecule), R denotes a methyl group or phenyl group, m denotes an integer 50-1000, and n denotes an integer 1-40.)

For the polyalkylene glycol-modified silicone of the present invention, commercial products can be used. Examples of the commercial product include FZ-2250 and Silstyle 401 (from Dow Corning Toray Company Ltd.).

The blend ratio of ingredient (a) is preferably 0.1-10 wt%, and more preferably 1-10 wt%, of the total amount of the hair cosmetic. If it is less than 0.1 wt%, then sufficiently moist texture and smoothness cannot be obtained. If it is over 10 wt%, then filminess is felt and stickiness and stiffness arise, resulting in a heavy and taut tactile sensation.
((b) Dimethylpolysiloxane having a viscosity of 100 cs or less)

Ingredient (b) used in the present invention dissolves the aforementioned ingredient (a); examples of commercially available products include TSF451-5A, TSF451-10A, TSF451-20A, and TSF451-100A (from Momentive Performance Materials, Inc.), SH200 C Fluid 5cs, SH200 C Fluid 6cs, SH200 C Fluid 10cs, SH200 C Fluid 20cs, SH200 C Fluid 30cs, SH200 C Fluid 50cs, and SH200 C Fluid 100cs (from Dow Corning Toray Company Ltd.), KF-96L-0.65cs, KF-96L-1cs, KF-96L-1. 5cs, KF-96L-2cs, KF-96L-5cs, KF-96A-6cs, KF-96-10cs KF-96-20cs KF-96-30cs KF-96-50cs, and KF-96-100cs (from Shin-Etsu Silicone Co., Ltd.).

The blend ratio of ingredient (b) is preferably 0.5-79 wt%, and more preferably 5-70 wt%, of the total amount of the hair cosmetic. If it is less than 0.5 wt%, then it is not sufficient for dissolving ingredient (a). If it is more than 79 wt%, then oily-squeakiness occurs and smoothness of the finish is no longer good.

The blend ration (mass ratio) between the aforementioned ingredient (a) and ingredient (b) is preferably (a) : (b) = 1 : 5 - 1 : 100, and more preferably (a) : (b) = 1 : 10 - 1 : 50. If ingredient (a) is more than 1/5 of ingredient (b), then stickiness arises and results in a heavy and taut tactile sensation. If ingredient (a) is less than 1/100 of ingredient (b), then oily-squeakiness occurs and a sufficient moisturizing sensation cannot be obtained.

### (Other oil components)

The present invention contains ingredient (a) and ingredient (b) as oil components. The following oils are examples of oil components other than ingredient (a) and ingredient (b) that are blended into the present invention.

Examples include isoparaffin hydrocarbons having a boiling point under normal pressures of 60-260° C; specific examples include Isopar A (registered trademark) from Exxon, as well as Isopar C, Isopar D, Isopar E, Isopar G, Isopar H, Isopar K, Isopar L, and Isopar M, and Shellzol 71 (registered trademark) from Shell, Solutol 100 (registered trademark), as well as Solutol 130 and Solutol 220, and also isododecane and isohexadecane.

The examples also include cyclic silicones such as octamethylcyclotetrasiloxane, decamethylcyclopentasiloxane, and dodecamethylcyclohexasiloxane.

The oil components further include the oil components generally used in cosmetics such as liquid fats and oils such as avocado oil, tsubaki oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japanese gimlet oil, jojoba oil, germ oil, triglycerin, glyceryl trioctanoate, and glyceryl triisopalmitate; solid fats and oils such as cacao butter, coconut oil, horse fat, hydrogenated coconut oil, palm oil, beef tallow, mutton tallow, hydrogenated beef tallow, palm kernel oil, lard, beef bone fat, Japanese core wax nucleus oil, hydrogenated oil, neatsfoot oil, Japanese core wax, and hydrogenated castor oil; waxes such as beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether; hydrocarbons such as liquid petrolatum, ozocerite, squalene, pristane, paraffin, ceresin, squalene, petrolatum, and microcrystalline wax; fatty acid oils; alcohols; and ester oils such as cetyl octanoate and isopropyl myristate.

The blend ratio of the total oil components including ingredient (a) and ingredient (b) is 50-80 wt%, more preferably 60-80 wt%. If the blend ratio of the oil components is less than 50 wt%, then the treatment effect becomes insufficient and the viscosity is hard to achieve, resulting in separation of the emulsion over time. If the blend ratio of the oil components is over 80 wt%, then too much thickening makes it hard to prepare a homogeneous emulsion and sometimes the mixture turns into the water-in-oil type. Also, the blend ratio of (a) + (b) is preferably 50 wt% or more of the total amount of the oil components.

Conventional oil-in-water hair cosmetics have less oil content than that of the present invention and highly viscous gel-like formulations have been made by thickening the water phase with water soluble polymers. However, in the present invention, the blend ratio of the oil components including high molecular weight silicone is as high as 50 wt% or more, resulting in more emulsified particles; therefore gel-like high viscosity can be maintained without adding water soluble thickeners as conventionally done and the emulsion stability is better. Also, the aforementioned high oil component content of the cosmetic of the present invention results in a higher hair treatment effect.
((c) Polyhydric alcohol)

Polyhydric alcohol is an ingredient necessary to keep the oil phase and water phase almost the same in terms of the refractive index so that the system becomes transparent-semi-transparent.

Examples of (c) polyhydric alcohol used in the present invention include one, two or more selected from glycerin, diglycerin, propylene glycol, dipropylene glycol, 1,3-butylene glycol, sorbitol, maltitol, xylitol, mannitol, and inositol.

The blend ratio of ingredient (c) is preferably 1-25 wt%, and more preferably 5-25 wt%, of the total amount of the hair cosmetic. If it is less than 5 wt%, then it is difficult to secure transparency. If it is over 25 wt%, then stickiness increases and the finish becomes heavy.

When there are less oil components, there is more water as a result, which has a lower refractive index, and therefore a large amount of polyhydric alcohol, which has a high refractive index, must be blended in to match the refractive index of the water phase with the refractive index of the oil phase. On the contrary, the present invention has a high blend ratio of the oil components and therefore transparency can be maintained even with a reduced amount of the polyhydric alcohol.
((d) Hydrophilic surfactant)

Examples of the (d) hydrophilic surfactant used in the present invention include cationic surfactants, anionic surfactants, ampholytic surfactants, and nonionic surfactants; specific examples follow. Of them, those having an HLB of 8 or higher are desirable for the purpose of the oil-in-water emulsification.

### Cationic surfactant

Examples include alkyltrimethylammonium salts (for example, stearyltrimethyl ammonium chloride, lauryltrimethyl ammonium chloride, and behenyltrimethyl ammonium chloride) alkylpyridinium salts (for example, cetylpyridinium chloride), distearyldimethylammonium chloride dialkyldimethylammonium salt; poly (N,N'-dimethyl-3,5-methylene piperidinium) chloride; alkyl quaternary ammonium salts; alkyl dimethylbenzyl ammonium salts; alkyl isoquinolinium salts; dialkyl morpholium salts; POE alkyl amines; amine salts (such as alkyl amine salts); polyamine fatty acid derivatives; amylalcohol fatty acid derivatives; fatty acid amidepropyl derivatives; benzalkonium chloride; and benzethonium chloride.

### Anionic surfactant

Examples include higher fatty acid salts such as sodium laurate and sodium palmitate, alkyl sulfates such as potassium lauryl sulfate, alkyl sulfonates, polyoxyethylene alkyl sulfonate, alkylbenzene sulfonate, N-acyl amino acid salts such as N-acyl sarcosinate and sodium stearoyl glutamate, α-olefin sulfonate, alkyl ether acetate, polyoxyethylene alkyl ether acetate, N-acyl-N-alkyl taurate such as N-acyl-N-methyl taurate.

### Ampholytic surfactant

Examples include the amide betaine type such as amide propyl betaine laurate and amide propyl betaine cocoate, the amidesulfobetaine type such as lauryl amidealkylenedimethyl aminosulfobetaine, the betaine type such as lauryl (dimethyl) betaine, stearyl (dimethyl) betaine, stearyldihydroxyethyl betaine, the sulfobetaine type such as lauryl sulfobetaine and laurylhydroxy sulfobetaine, sodium 2-undecyl-N, N, N-(hydroxyethyl carboxymethyl)-2-imidazoline, 2-cocoyl-2-imidazolinium hydroxide-1-carboxyethyloxy 2 sodium salt, and cocoyl alkyl N-carboxyethyl N-hydroxyethyl imidazolinium betaine.

### Nonionic surfactant

Examples include sorbitan fatty acid esters such as sorbitan monostearate and sorbitan sesquioleate, alkylene glycol fatty acid esters such as diethylene glycol laurate, propylene glycol laurate, ethylene glycol monooleate, and ethylene glycol distearate, hydrogenated castor oil derivatives, glycerin alkyl ether, POE sorbitan fatty acid esters such as POE sorbitan monooleate and polyoxyethylene sorbitan monostearate, POE sorbit fatty acid esters such as POE-sorbit monolaurate, POE glycerin fatty acid esters such as POE-glycerin monoisostearate, POE glycerin fatty acid esters such as polyethylene glycol monooleate and POE distearate, POE alkyl ethers such as POE-octyldodecyl ether, POE alkylphenyl ethers such as POE nonylphenyl ether, POE-POP alkyl ethers, pluaronic types, POE castor oil, POE hydrogenated castor oil derivatives, sugars such as sugar esters, sugar ethers, and sugar amides, and alkyl glycosides.

The blend ratio of (d) hydrophilic surfactant is 1-10 wt%, preferably 2-5 wt%. If the blend ratio of the hydrophilic surfactant is less than 1 wt%, then it becomes difficult to ensure the long term stability of the emulsified product. If the blend ratio is over 10 wt%, then stickiness increases and the finish becomes heavier.

### (Water soluble polymer thickener)

In the present invention, it is preferable not to blend in a water soluble thickener, or to blend it in to have 0.5 wt% or less. If the blend ratio is over 0.5 wt%, then tautness and stiffness increase, resulting in a poor tactile sensation.

The following are examples of the water soluble polymer thickener.

### Polysaccharide

Examples include gum arabic, glucan, succinoglycan, carrageenan, karaya gum, gum tragacanth, guar gum, locust bean gum, galactomannan gum, xanthan gum, starch, carob gum, quince seed (Cydonia oblonga), casein, dextrin, gelatin, sodium pectate, sodium arginate, methyl cellulose, ethyl cellulose, carboxymethyl cellulose, hydroxyethyl cellulose, hydroxypropyl cellulose, crystal cellulose, curdlan, O-[2-hydroxy-3-(trimethylammonio) propyl] hydroxyethyl cellulose chloride, 0-[2-hydroxy-3-(trimethylammonio) propyl] guar gum chloride, O-[2-hydroxy-3-(trimethylammonio) propyl] locust bean gum chloride, and hydroxypropyltrimonium chloride.

### Acrylic and vinyl types

Anionic examples include alkyl acrylate/diacetone acryl amide copolymer (such as Plus Size L-53P, Plus Size L-9909B, and Plus Size L-9948B from Goo Chemical Co. Ltd.), alkyl acrylate/octylacryl amide copolymer (Dermacryl 79 from AczoNobel), polyethylene glycol/polypropylene glycol-25/dimethicone/acrylates copolymer (Rubiflex SILK from BASF), acrylic acid/amide acrylate/acrylamide/ethyl acrylate copolymer (Ultrahold 8 and Ultrahold Strong from BASF), and alkyl acrylate copolymer (Aniset NF-1000 and Aniset HS-3000 from Osaka Organic Chemical Industry Ltd.).

Ampholytic examples include octyl acrylamide/hydroxypropylpropyl acrylate/butylaminoethyl methacrylate copolymer (Amphomer SH30 and Amphomer LV-71 from AkzoNobel), methacryloyloxyethyl carboxybetaine/alkyl methacrylate copolymer (such as Yukaformer R205, Yukaformer 301, Yukaformer SM, and Yukaformer 104D from Mitsubishi Chemical, and RAM Resin-1000, RAM Resin-2000, RAM Resin-3000, and RAM Resin-4000 from Osaka Organic Chemical Industry Ltd.), dimethylallyl ammonium chloride/acrylic acid copolymer (Marcoat 280 and Marcoat 295 from Nalco), and dimethylallyl ammonium chloride/acrylamide/acrylic acid copolymer (Marcoat 3330 and Marcoat 3331 from Nalco).

Cationic examples include vinyl pyrrolidone/dimethylaminoethyl methacrylate copolymer diethyl sulfate (H.C. Polymer 1S(M) and H.C. Polymer 2 from Osaka Organic Chemical Industry Ltd.), vinyl pyrrolidone/dimethylaminopropyl methcarylamide/lauryldimethylaminopropyl methacrylamide copolymer (Styleze W-20 from ISP), vinyl pyrrolidone/N,N-dimethylaminoethyl methacrylate/alkyl acrylate/tripropylene glycol copolymer (Cosquat GA 467 and Cosquat GA 468 from Osaka Organic Chemical Industry Ltd.), poly-dimethylmethylenepiperidinium chloride (Marcoat 100 from Nalco), dimethyldiallyl ammonium chloride/acrylamide copolymer (Marcoat 550 from Nalco), trimethylaminopropyl acrylamide/dimethyl acrylamide copolymer, and Japanese Patent Laid-Open 2008-189582 bulletin.

Nonionic examples include polyvinyl pyrrolidone (Luviskol K17, Luviskol K30, and Luviskol K90 from BASF), PVP K from ISP, vinyl pyrrolidone/vinyl acetate copolymer (PVP/VA S-630, PVP/VA E-735, and PVP/VA E-335 from ISP, Luviskol VA73W and Luviskol 37E from BASF, PVA-6450 from Osaka Organic Chemical Industry Ltd., vinylmethyl ether/alkyl malate copolymer (Gantrez A-425, Gantrez ES-225, and Gantrez ES-335 from ISP), and vinyl pyrrolidone/methacrylamide/vinyl imidazole copolymer (Luviset Clear from BASF).

### Urethane type

Examples include Yodozol PUD from AkzoNobel, Luviset P.U.R. from BASF, the polymer of Japanese Patent Laid-Open 2006-213706 bulletin, and, for the acryl-urethane type, Dynam X from AkzoNobel.

Various ingredients other than the aforementioned which are usually used in emulsified compositions for cosmetics can be blended into the hair cosmetic of the present invention as appropriate for the purpose and within the range that does not adversely affect the effects of the present invention. Such ingredients include humectants such as hyaluronic acid, chondroitin sulfuric acid, and pyrrolidone carboxylate, ultraviolet absorbents, and ultraviolet scattering agents.

Also included are proteins or hydrolyzed proteins such as soy bean protein, gelatin, collagen, silk fibroin, and elastin, preservatives such as ethylparaben and butylparaben, activators such as various amino acids, biotin, and pantothenic acid derivatives, anti-seborrhea agents such as *γ*-oryzanol, sodium dextran sulfate, and vitamin E, diluting agents such as ethanol, isopropanol, and tetrachlorodifluoroethane, thickeners such as carboxyvinyl polymer, drugs, perfumes, and coloring agents; they can be blended in as appropriate and needed.

The hair cosmetic of the present invention, despite the fact that its external appearance is transparent or semi-transparent, is the oil-in-water emulsified type that is gel-like and rich in the oil components. Therefore, in spite of its transparent and aesthetic external appearance, it is free of stickiness such as is found in oil based cosmetics, absorbs well in the hair, does not drip at the time of use, and manifests good usability such as absorption and spreadability at the time of application.

In the present invention, "transparent or semi-transparent" means that the L value is 40 or higher when measured with a color-difference meter (spectro color meter SE2000 from Nippon Denshoku).

The cosmetic of the present invention, in terms of the product form, can be used as a hair rinse, hair conditioner, hair pack, hair cream, hair treatment, etc., and, in terms of the manner of use, it can be a type that is not rinsed away after application to the hair or a type that is rinsed away after application to the hair, the former type being more preferable.

### EXAMPLES

The present invention is described in detail below by referring to Examples. The present invention is not limited to these Examples. The blend ratios are in mass-percentage units unless specified otherwise.

Before going into Examples, the evaluation method and the evaluation criteria used in the present invention are described.

### (1) External appearance (stability and transparency)

The prepared samples were put into a transparent glass container (screw tube), and, after being left still at ordinary temperatures for one month, the samples were checked for their homogeneity. For the homogeneous ones, a color-difference meter (Spectro color meter SE2000 from Nippon Denshoku) was used to measure the L value, and the evaluation was made by defining those having an L value of 40 or higher as transparent.

### <Evaluation criteria>

A: The L value measured as describe above is 40 or higher.
D: The L value measured as describe above is less than 40.

### (2) Spreadability at the time of application

A panel of ten female specialists put 0.5 g of the sample on their palms and spread it with their fingers, and evaluated the spreadability by means of a sensory test. Each of them graded the samples based on the following evaluation criteria; the points from the ten were totaled and each test item was evaluated based on the following evaluation criteria.

### <Evaluation points criteria>

5 points: Spreads well
4 points: Spreads relatively well
3 points: Midway
2 points: Spreads relatively poorly
1 point: Spreads poorly

### <Evaluation criteria>

A: The total score is 40 points or higher.
B: The total score is 30 points or more and less than 40 points.
C: The total score is 20 points or more and less than 30 points.
D: The total score is less than 20 points.

### (3) Non-drippiness

A panel of ten female specialists put 0.5 g of the sample on their palms and spread it with their fingers, and evaluated the lack of dripping through their fingers by means of a sensory test. Each of them graded the samples based on the following evaluation criteria; the points from the ten were totaled and each test item was evaluated based on the following evaluation criteria.

### <Evaluation point criteria>

5 points: Not drippy
4 points: Relatively not drippy
3 points: Midway
2 points: Relatively drippy
1 point: Drippy

### <Evaluation criteria>

A: The total score is 40 points or higher.
B: The total score is 30 points or more and less than 40 points.
C: The total score is 20 points or more and less than 30 points.
D: The total score is less than 20 points.

### (4) Absorption at the time of application

0.5 g of the sample was applied on one bundle of black virgin hair (length 20 cm, weight 4 g) and rubbed in with fingers; the degree of absorption was evaluated by a panel of ten female specialists by means of a sensory test. The evaluation criteria are as follows:

### <Evaluation point criteria>

5 points: Absorbs well
4 points: Absorbs relatively well
3 points: Midway
2 points: Absorbs relatively poorly
1 point: Absorbs poorly

### <Evaluation criteria>

A: The total score is 40 points or higher.
B: The total score is 30 points or more and less than 40 points.
C: The total score is 20 points or more and less than 30 points.
D: The total score is less than 20 points.

### (5) Non-stickiness at the time of application

0.5 g of the sample was applied on one bundle of black virgin hair (length 20 cm, weight 4 g) and rubbed in with fingers; the non-stickiness of the hair was evaluated by a panel of ten female specialists by means of a sensory test. The evaluation criteria are as follows:

### <Evaluation point criteria>

5 points: Not sticky
4 points: Relatively not sticky
3 Points: Midway (cannot say either way)
2 points: Relatively sticky
1 point: Sticky

### <Evaluation criteria>

A: The total score is 40 points or higher.
B: The total score is 30 points or more and less than 40 points.
C: The total score is 20 points or more and less than 30 points.
D: The total score is less than 20 points.

### (6) Smoothness of the finish

0.5 g of the sample was applied on one bundle of black virgin hair (length 20 cm, weight 4 g) and rubbed in with fingers for finish; the smoothness of the hair surface was evaluated by a panel of ten female specialists by means of a sensory test. The evaluation criteria are as follows:

### <Evaluation point criteria>

5 points: Very smooth
4 points: Smooth
3 Points: Midway (cannot say either way)
2 points: Relatively not smooth
1 point: Not smooth

### <Evaluation criteria>

A: The total score is 40 points or higher.
B: The total score is 30 points or more and less than 40 points.
C: The total score is 20 points or more and less than 30 points.
D: The total score is less than 20 points.

### (7) Sensation of running fingers through the hair after finish

0.5 g of the sample was applied on one bundle of black virgin hair (length 20 cm, weight 4 g) and rubbed in with fingers for finish; the sensation of running fingers through the hair after finish was evaluated by a panel of ten female specialists by means of a sensory test. The evaluation criteria are as follows:

### <Evaluation point criteria>

5 points: Sensation of running fingers through the hair after finish is very good.
4 points: Sensation of running fingers through the hair after finish is good.
3 Points: Midway (cannot say either way)
2 points: Sensation of running fingers through the hair after finish is relatively poor.
1 point: Sensation of running fingers through the hair after finish is poor.

### <Evaluation criteria>

A: The total score is 40 points or higher.
B: The total score is 30 points or more and less than 40 points.
C: The total score is 20 points or more and less than 30 points.
D: The total score is less than 20 points.

### Examples 1-11, Comparative examples 1-4

Hair cosmetics composed of the recipe ingredients shown in the following Tables 1 and 2 were prepared with the method described below. For the obtained hair cosmetics, the aforementioned criteria were used to evaluate the external appearance, spreadability at the time of application, non-drippiness, absorption at the time of application, non-stickiness at the time of application, smoothness of the finish, and the sensation of running fingers through the hair after finish is good. The results are also shown in Tables 1 and 2.

In the present invention, both good absorption and spreadability on the hair surface and the hair treatment effect have been achieved by having a high blend ratio of the oil components containing high polymer silicone in an oil-in-water emulsified cosmetic.

### <Preparation method>

(5) was heated and melted, and then dissolved in (4), to which an aqueous solution prepared by putting (2) into (1) followed by stirring and dispersing was added, followed by homogeneous mixing. (3) is additionally added to make the water phase part. The oil phase part was prepared by mixing (6), (7), (8), (9), (10), (11), (12), (13), and (14) and added to the water phase part, followed by emulsification with a homomixer to obtain a transparent hair cosmetic (hair treatment that would not be rinsed away).

**{Table 1}**

| | Example 1 | Exampl e 2 | Example 3 | Example 4 | Example 5 | Example 6 | Example 7 | Example 8 | Example 9 | Example 10 | Example 11 |
|---|---|---|---|---|---|---|---|---|---|---|---|
| (1) Ion-exchanged water | Balance | Balance | Balance | Balance | Balance | Balance | Balance | Balance | Balance | Balance | Balance |
| (2) Carboxyvinyl polymer (*1) | 0.1 | 0.1 | 0.1 | 0.1 | 0.1 | 0.1 | 0.1 | 0.1 | 0.1 | 0.1 | - |
| (3) 2-ami no-2-methyl propano l | 0.04 | 0.04 | 0.04 | 0.04 | 0.04 | 0.04 | 0.04 | 0.04 | 0.04 | 0.04 | 0.04 |
| (4) 1,3-butylene glycol | 20 | 20 | 20 | 20 | 22 | 22 | 23 | 19 | 10 | 23 | 20 |
| (5) P0E (60) hydrogenated castor oil | 3 | 3 | 3 | 3 | 3 | 3 | 3 | 3 | 3 | 3 | 3 |
| (6) Highly polymerized silicone (*2) | 10 | - | - | - | - | 10 | 33.3 | 2 | 15 | | 10 |
| (7) Highly polynerized silicone with both ends hydroxy-modified (*3) | - | 15 | - | - | - | - | - | - | - | 0.5 | - |
| (8) Highly polymerized amino-modified silicone (*4) | - | - | 15 | - | - | - | - | - | - | - | - |
| (9) Highly polarized amomium-modified si licone (*5) | - | - | - | 15 | - | - | - | - | - | - | - |
| (10) Highly polymerized polyalkylene glycol-modified silicone (*6) | - | - | - | | 8.6 | - | - | - | - | - | - |
| (11) Low viscosity silicone (*7) | 50 | 45 | 45 | 45 | 51.4 | - | 26.7 | 58 | 65 | 0.5 | 50 |
| (12) Low viscosity silicone (*8) | - | - | - | - | - | 50 | - | - | - | - | - |
| (13) Isanonyl isononanoate | - | - | - | - | - | - | - | - | - | 5 | - |
| (14) Decamethylcyclopentasiloxane | - | - | - | - | - | - | - | - | - | 55 | - |
| Total | 100 | 100 | 100 | 100 | 100 | 100 | 100 | 100 | 100 | 100 | 100 |
| Total oil phase (wt%) | 60 | 60 | 60 | 60 | 60 | 60 | 60 | 60 | 80 | 61 | 60 |
| (a)/(b) (weight ratio) | 1/19 | 1/15 | 1/19 | 1/39 | 1/17.1 | 1/19 | 1/5 | 1/99 | 1/16.8 | 1/5 | 1/19 |
| Viscosity (mPa·s) | 15600 | 14800 | 21000 | 28500 | 15200 | 16100 | 15800 | 16100 | 165000 | 15800 | 8200 |
| (1) External appearance | A | A | A | A | A | A | A | A | A | A | A |
| (2) SpreadabiI ity at the time of application | A | A | A | A | A | A | A | A | B | A | A |
| (3) Non-drippiness | A | A | A | A | A | A | A | A | A | A | A |
| (4) Absorption at the time of application | A | A | A | A | A | A | A | A | A | A | A |
| (5) Non-Stickiness at the time of application | A | A | A | A | A | A | B | A | A | A | A |
| (6) Smothness of the finish | A | A | A | A | A | A | A | B | A | B | A |
| (7) Sensation of running fingers through the hair after finish | A | A | A | A | A | B | B | A | A | B | A |

**{Table 2}**

| | Comparative example 1 | Comparative example 2 | Comparative example 3 | Comparative example 4 |
|---|---|---|---|---|
| (1) Ion-exchanged water | Balance | Balance | Balance | Balance |
| (2) Carboxyvinyl polymer (*1) | 0.1 | 0.1 | 0.1 | 0.1 |
| (3) 2-amino-2-methylpropanol | 0.04 | 0.04 | 0.04 | 0.04 |
| (4) 1,3-butylene glycol | 18 | 19 | 27 | 20 |
| (5) POE (60) hydrogenated castor oil | 3 | 3 | 3 | 3 |
| (6) Highly polymerized silicone (*2) | - | 60 | 10 | 10 |
| (11) Low viscosity silicone (*7) | 60 | - | 40 | 30 |
| Total | 100 | 100 | 100 | 100 |
| Total oil phase (wt%) | 60 | 60 | 50 | 40 |
| (a)/(b) (weight ratio) | 0 | 1/2.33 | 1/15.7 | 1/12.3 |
| Viscosity (mPa·s) | 17000 | 16500 | 18200 | 13000 |
| (1) External appearance | A | A | A | D |
| (2) Spreadability at the time of appl ication | A | A | A | A |
| (3) Non-dr ippiness | A | A | A | A |
| (4) Absorption at the time of application | B | A | A | A |
| (5) Non-stickiness art the time of application | A | C | D | C |
| (6) Smoothness of the finish | C | A | C | A |
| (7) Sensation of running fingers through the hair after finish | A | C | B | B |

| | | | | |
|---|---|---|---|---|
| *1: Hibis Wako 105 (from Wako Pure Chemical Industries, Ltd.) *2: BY11-206 (30% highly polymerized silicone/70% low viscosity silicone (20 cs) (from Dow Corning Toray Company Ltd.) *3: XF-C2520 (20% Highly polymerized silicone with both ends hydroxy-modified/80% cyclopentasiloxane (from Momentive Performance Materials Japan LLC) *4: Amino-modified silicone (20%)/80% low viscosity silicone (20 cs) wherein the amino-modified silicone is represented by the following general formula (4) where R¹ = methyl group, = (CH₂)₃N(CH₃)(CH₂)₂N(CN₃)₂, m = 5000, n = 150 | | | | |

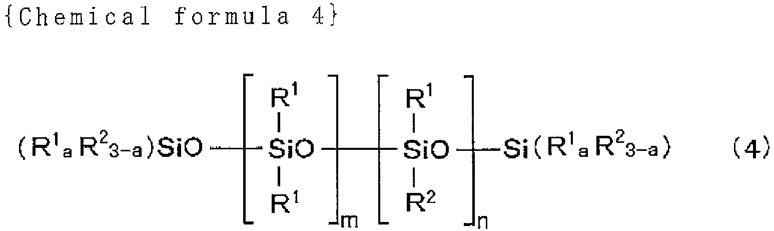

*5: Ammonium-modified polymer silicone (10%)/ 90% low viscosity silicone (20 cs) wherein the ammonium-modified polymer silicone is represented by said general formula (2) where in R¹ = methyl group, = hydroxyl group, R³ = -(CH₂)₃N⁺ (CH₃)₃Cl⁻, m = 18,000, and n = 2.
*6: FZ-2250 (35% polyalkylene glycol-modified highly polymerized silicone/65% volatile isoparaffin) (from Dow Corning Toray Company Ltd.)
*7: KF-96A-6cs (Shin-Etsu Chemical Co., Ltd.)
*8: KF-96A-100cs (Shin-Etsu Chemical Co., Ltd.)

Formulation examples of the oil-in-water hair cosmetic of the present invention are shown below. Needless to say, the present invention is not limited at all by these formulation examples and it is specified by the scope of the claim.

Formulation example 1 (Hair treatment that would not be rinsed away)

| Ingredients | (wt%) |
|---|---|
| (1) Ion-exchanged water | Balance |
| (2) Cationized cellulose | 0.2 |
| (Polymer JR-400 (from Union Carbide Japan)) | |
| (3) Glycerin | 5.0 |
| (4) Propylene glycol | 20.0 |
| (5) Polyoxyethylene (40) hydrogenated castor oil | 2.5 |
| (6) Stearyltrimethylammonium chloride (25% aqueous solution) | 1.0 |
| (7) Hydrolyzed wheat protein solution | 0.5 |
| (Cropeptide W (from Croda Inc.)) | |
| (8) Amino-modified silicone | 10.0 |
| (Amino-modified silicone (20%)/80% low viscosity silicone (20 cs) where in the amino-modified silicone is represented by the following general formula (4) where R¹ = methyl group, R² = -(CH₂)₃N(CH₃)₂, m = 5000, n = 20) | |
| (9) Low viscosity silicone | 45.0 |
| (KF-96-20cs (from Shin-Etsu Chemical Co.,Ltd.)) | |
| (10) Poly (oxyethylene/oxypropylene)/ methylpolysiloxane copolymer | 5.0 |
| (FZ-2250 (from Dow Corning Toray Company Ltd.)) | |
| (11) Perfume | Appropriate amount |

### <Preparation method)

(5) was heated and melted, and then dissolved in (4), to which (2) was added and homogeneously dispersed, and then (11), (1), (3), (6), and (7) were added in this order and mixed to obtain the water phase part. The oil phase part was prepared by mixing (8), (9), and (10) and added to the water phase part, followed by emulsification with a homomixer to obtain a transparent hair cosmetic (hair treatment that would not be rinsed away).

### Formulation example 2 (Hair treatment that would not be rinsed away)

| Ingredients | (wt%) |
|---|---|
| (1) Ion-exchanged water | 0.5 |
| (2) Acrylic acid/alkyl acrylate (C10-30) copolymer | 0. 2 |
| (Pemulen TR-2 (from B. F. Goodrich Chemical | |
| Company)) | |
| (3) Polyoxyethylene (10) methyl glucoside | |
| (Glucam E-10 (from Lubrizol Japan Limited)) 10.0 | |
| (4) Propylene glycol | 10.0 |
| (5) Sodium cocoampho acetate | 2.0 |
| (6) Isostearic acid | 1.5 |
| (7) Soy lecithin | 0.5 |
| (8) Silicone with both ends hydroxy-modified | 20 |
| (XF49-C2070 (from Momentive Performance Materials Japan)) | |
| (9) Low viscosity silicone | 10.0 |
| (KF-96-100cs (from Shin-Etsu Chemical Co., Ltd. )) | |
| (10) Isododecane | 20.0 |
| (11) Perfume | Appropriate amount |
| (12) Triethanolamine | 0. 1 |

### <Preparation method>

(2) and (7) were homogeneously dispersed in (1), to which (3), (5), and (12) were added in this order, and after homogeneous stirring, (11) dissolved in (4) was added to obtain the water phase part. The oil phase part was prepared by mixing (6), (8), (9), and (10) and added to the water phase part, followed by emulsification with a homomixer to obtain a transparent hair cosmetic (hair treatment that would not be rinsed away).

### Formulation example 3 (Hair treatment that would not be rinsed away)

| Ingredients | (wt%) |
|---|---|
| (1) Ion-exchanged water | Balance |
| (2) 20% aqueous solution of vinyl pyrrolidone /dimethylaminoethyl methacrylate copolymer diethyl sulfate (Gafquart 755N from ISP) | 2.0 |
| (3) Sorbitol | 4.0 |
| (4) Propylene glycol | 10.0 |
| (5) Polyoxyethylene (20) cetyl ether | 3.0 |
| (6) Dicocoylethyl hydroxyethylmonium methosulfate (Dehyquart L80 (from Cognis Japan) | 0.5 |
| (7) Hydroxyethyl urea | 1.0 |
| (8) Amino-modified silicone | 14.0 |
| (Amino-modified silicone (20%)/80% low viscosity silicone (20 cs) wherein the amino-modified silicone is represented by the following general formula (4) where R¹ = methyl group, = -(CH₂)₃N(CH₃) 2, m = 10000, n = 20) | |
| (9) Low viscosity silicone | 55.0 |
| (KF-96-6cs (from Shin-Etsu Chemical Co., Ltd.)) | |
| (10) Silicone elastomer | 1.0 |
| (DC9041 Silicone Elastomer Blend (from Dow Corning Toray Company Ltd.)) | |
| (11) Perfume | Appropriate amount |
| (12) Amino acid 50% aqueous solution | 0.5 |
| (Prodew 500 (from Ajinomoto Co., Inc.)) | |

### <Preparation method>

(5) was melted, and then added to (4), to which (11) was added and homogeneously dispersed, and then (1), (3), (6), (7), and (12) were added in this order and mixed to obtain the water phase part. The oil phase part was prepared by mixing (8), (9), and (10) and added to the water phase part, followed by emulsification with a homomixer to obtain a transparent hair cosmetic (hair treatment that would not be rinsed away).

### Formulation example 4 (Hair treatment that would not be rinsed away)

| Ingredients | (wt%) |
|---|---|
| (1) Ion-exchanged water | Balance |
| (2) Vinyl pyrrolidone/N,N-dimethylaminoethyl methacrylate/alkyl acrylate/tripropylene glycol copolymer | 0.2 |
| (Cosquat GA467 (from Osaka Organic Chemical Industry Ltd.)) | |
| (3) Diglycerin | 5.0 |
| (4) Propylene glycol | 20.0 |
| (5) Glyceryl polyethylene glycol stearate (5) | 2. 5 |
| (6) Polyoxyethylene (10) methyl ether dimethicone | 10.0 |
| (SH-3749 (from Dow Corning Toray Company Ltd.)) | |
| (7) L-menthol | 0.2 |
| (8) Highly polymerized silicone | 10.0 |
| (BY11-206 (30% highly polymerized silicone/70% low viscosity silicone (20 cs) ) | |
| (9) Low viscosity silicone | 38.0 |
| (KF-96-6cs (from Shin-Etsu Chemical Co., Ltd.)) | |
| (10) Methylphenylsiloxane | 2.0 |
| (KF-56 (from Shin-Etsu Chemical Co., Ltd.)) | |
| (11) Perfume | Appropriate amount |
| (12) Lactic acid | 0.1 |

### <Preparation method>

(5) was melted, and then added to (4), to which (11) was added and homogeneously dispersed, and then (1), (3), (6), (7), and (12) were added in this order and mixed to obtain the water phase part. The oil phase part was prepared by mixing (8), (9), and (10) and added to the water phase part, followed by emulsification with a homomixer to obtain a transparent hair cosmetic (hair treatment that would not be rinsed away).

### Formulation example 5 (hair conditioner)

| Ingredients | (wt%) |
|---|---|
| (1) Ion-exchanged water | Balance |
| (2) Polyoxyethylene decyltetradecyl ether (20E0)/hexamethylene diisocyanate/polyoxyethylene glycol (240EG) copolymer | 0.5 |
| (3) Polyethylene glycol (molecular weight 400) | 8. 0 |
| (4) Dipropylene glycol | 10.0 |
| (5) Polyoxyethylene (20) cetyl ether | 2.5 |
| (6) Stearoxy hydroxy propylamine | 2.0 |
| (7) Cationized oligosaccharide | 0.5 |
| (Oligoquat M from GSI Creos Corporation) | |
| (8) Highly polymerized silicone | 20.0 |
| ( BY11-206 (30% highly polymerized silicone/70% low viscosity silicone (20 cs) ) | |
| (9) Low viscosity silicone | 50.0 |
| (KF-96-6cs (from Shin-Etsu Chemical Co., Ltd.)) | |
| (10) Bisisobutyl PEG-14/amodimethicone copolymer | |
| | 0. 5 |
| (Silstyle 104 (from Dow Corning Toray Company Ltd.)) | |
| (11) Perfume | Appropriate amount |
| (12) L-glutamic acid | 0.6 |

### <Preparation method)

Melted (5) as well as (2), (6), and (11) were added to heated (4), to which (1), (3), (7), and (12) were added in this order and mixed to obtain the water phase part. The oil phase part was prepared by mixing (8), (9), and (10) and added to the water phase part, followed by emulsification with a homomixer to obtain a transparent hair cosmetic (hair conditioner).

### Formulation example 6 (hair conditioner)

| Ingredients | (wt%) |
|---|---|
| (1) Ion-exchanged water | Balance |
| (2) Cationized guar gum | 0.2 |
| (Katinal CG-100S (from Toho Chemical Industry Co., Ltd.)) | |
| (3) Polyethylene glycol (molecular weight 4 million) | 0.1 |
| (4) Dipropylene glycol | 17.0 |
| (5) Polyoxyethylene (20) cetyl ether | 2.5 |
| (6) Behenyl trimethyl ammonium chloride | 2.0 |
| (7) Propyltrimonium chloride acrylamide (DMAPAAC)/dimethylacrylamide (DMAA) copolymer | 0. 2 |
| (DMAPAAC/DMAA = 30/70 (mole percentage), weight average molecular weight = 430,000) | |
| (8) Highly polymerized silicone | 20.0 |
| ( BY11-206 (30% highly polymerized silicone/70% low viscosity silicone (20 cs)) | |
| (9) Low viscosity silicone | 40.0 |
| (KF-96-6cs (from Shin-Etsu Chemical Co., Ltd.)) | |
| (10) Bis (C13-15 alkoxy) PG amodimethicone copolymer | 1.0 |
| (JP-8500 conditioning agent (from Dow Corning Toray Company Ltd.)) | |
| (11) Perfume | Appropriate amount |
| (12) Wine extract | 0.5 |

### <Preparation method>

Melted (5) as well as (2) and (6) were added to (4), to which (1), (3), (4), (7), and (12) were added in this order and mixed to obtain the water phase part. The oil phase part was prepared by mixing (8), (9), and (10) and added to the water phase part, followed by emulsification with a homomixer to obtain a transparent hair cosmetic (hair conditioner).

## Claims

1. A transparent or semi-transparent oil-in-water emulsified hair cosmetic characteristically comprising the following (a)-(d) wherein the blend ratio of the oil phase, including (a) and (b), is 50-80 wt% and the blend ratio (mass ratio) between (a) and (b) is (a) : (b) = 1 : 5 - 1 : 100.
(a) One, two or more silicones chosen from high polymer silicone, silicone with both ends hydroxy-modified, amino-modified silicone, ammonium-modified silicone, and polyalkylene glycol-modified silicone that are gum-like or have a viscosity of 1 million mPa·s (cs) or higher; 0.1-10 wt%
(b) Dimethylpolysiloxane having a viscosity of 100 mPa·s (cs) or less; 0.5-79 wt%
(c) Polyhydric alcohol; 1-25 wt%
(d) Hydrophilie surfactant; 1-10 wt%,
wherein transparent or semi-transparent means that the L value is 40 or higher when measured with a color-difference meter.

2. The oil-in-water emulsified hair cosmetic of claim 1 wherein the blend ratio of the oil phase, including (a) and (b), is 60-80 wt%.

3. The oil-in-water emulsified hair cosmetic of claim 1 wherein a water soluble polymer thickener is not blended in or the blend ratio thereof is 0.5 wt% or less.

4. The oil-in-water emulsified hair cosmetic of any one of claims 1 to 3, wherein the high polymer silicone of (a) is represented by the following general formula (1), 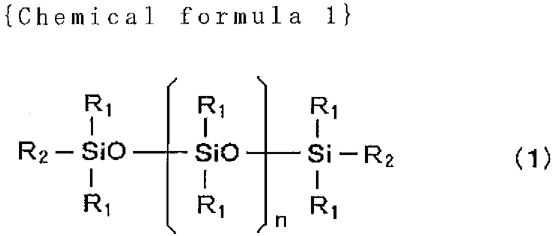 wherein R₁ denotes a methyl group or partially a phenyl group, and R₂ denotes a methyl or hydroxyl group, n denotes an integer 3,000-20,000.

5. The oil-in-water emulsified hair cosmetic of any one of claims 1 to 3, wherein amino-modified silicone and ammonium-modified silicone of (a) are represented by the following formula (2), 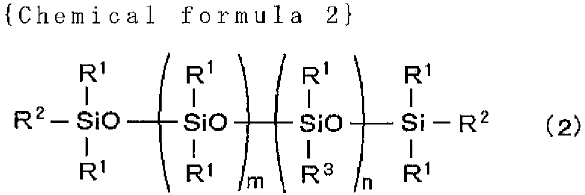 wherein R¹ denotes a methyl group or partially a phenyl group, and is the same as R³ or denotes a methyl or hydroxyl group, R³ denotes a substituent having an amino group or an ammonium group represented by formula R⁴Z, wherein R⁴ denotes a divalent alkylene group having 3 to 6 carbon atoms, Z denotes a monovalent group chosen from a group consisting of -NR⁵₂, -N⁺R⁵₃A⁻,
-NR⁵(CH₂)ₐNR⁵₂, -NR⁵(CH₂)ₐN⁺R⁵₃A⁻ and
-NR⁵(CH₂)ₐN(R⁵)C=O(R⁶), R⁵ denotes hydrogen or an alkyl group having a hydrogen or 1-4 carbon atoms, R⁶ denotes an alkyl group having 1-4 carbon atoms, A denotes C1, Br, or I, and a denotes an integer 2-6, m and n each denotes a positive integer, m + n denotes an integer 3,000-20,000, and n/m is 1/500-1/10,000.

6. The oil-in-water emulsified hair cosmetic of any one of claims 1 to 3, wherein the polyalkylene glycol-modified silicone of (a) is represented by the following general formula (3), 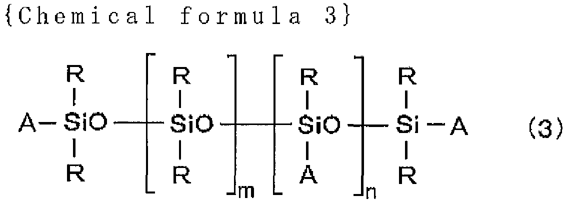 wherein R denotes a methyl group or phenyl group, m denotes an integer 50-1000, and n denotes an integer 1-40, A denotes a group selected from a group consisting of a methyl group, phenyl group, and polyoxyalkylene group represented by a general formula -C₃H₆O(C₂H₄O) a (C₃H₆O)bR', wherein in this formula R' is a group selected from a group consisting of hydrogen atom, acyl group, and alkyl group having 1-4 carbon atoms, and a and b each is an integer 5-50.

7. The oil-in-water emulsified hair cosmetic of any one of claims 1 to 6, wherein the blend ratio (mass ratio) between (a) and (b) is (a) : (b) = 1 : 10 - 1 : 50.

8. The oil-in-water emulsified hair cosmetic of any one of claims 1 to 7, further comprising other oil components other than (a) and (b) selected from isoparaffin hydrocarbons having a boiling point under normal pressures of 60-260° C, cyclic silicones such as octamethylcyclotetrasiloxane, decamethylcyclopentasiloxane, and dodecamethylcyclohexasiloxane, liquid fats and oils such as avocado oil, tsubaki oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese gimlet oil, Japanese gimlet oil, jojoba oil, germ oil, triglycerin, glyceryl trioctanoate, and glyceryl triisopalmitate; solid fats and oils such as cacao butter, coconut oil, horse fat, hydrogenated coconut oil, palm oil, beef tallow, mutton tallow, hydrogenated beef tallow, palm kernel oil, lard, beef bone fat, Japanese core wax nucleus oil, hydrogenated oil, neatsfoot oil, Japanese core wax, and hydrogenated castor oil; waxes such as beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether; hydrocarbons such as liquid petrolatum, ozocerite, squalene, pristane, paraffin, ceresin, squalene, petrolatum, and microcrystalline wax; fatty acid oils; alcohols; and ester oils such as cetyl octanoate and isopropyl myristate.

9. The oil-in-water emulsified hair cosmetic of any one of claims 1 to 8, wherein the polyhydric alcohol (c) comprises one, two or more selected from glycerin, diglycerin, propylene glycol, dipropylene glycol, 1,3-butylene glycol, sorbitol, maltitol, xylitol, mannitol, and inositol.

10. The oil-in-water emulsified hair cosmetic of any one of claims 1 to 9, wherein the hydrophilic surfactant (d) includes cationic surfactants, anionic surfactants, ampholytic surfactants, and nonionic surfactants.

11. The oil-in-water emulsified hair cosmetic of claim 10, wherein the hydrophilic surfactant (d) has an HLB of 8 or higher.

## Patentansprüche

1. Transparentes oder semi-transparentes Öl-in-Wasser-emulgiertes Haarkosmetikum, charakteristischerweise umfassend die folgenden (a)-(d), wobei das Mischungsverhältnis der Ölphase einschließlich (a) und (b) 50-80 Gew.-% und das Mischungsverhältnis (Massenverhältnis) zwischen (a) und (b), das heißt (a) : (b) = 1 : 5 - 1 : 100 beträgt,
(a) ein, zwei oder mehr Silicone, gewählt aus Hochpolymersilicon, Silicon, dessen beide Enden hydroxymodifiziert sind, aminomodifiziertes Silicon, ammoniummodifiziertes Silicon und Polyalkylenglykol-modifiziertes Silicon, welche gummiähnlich sind oder eine Viskosität von 1 Million mPa · s (cs) oder höher aufweisen; 0,1 - 10 Gew.-%,
(b) Dimethylpolysiloxan mit einer Viskosität von 100 mPa · s (cs) oder weniger; 0,5 - 79 Gew.-%
(c) mehrwertiger Alkohol; 1 - 25 Gew.-%
(d) hydrophiles Tensid; 1 - 10 Gew.-%,
wobei transparent oder semi-transparent bedeutet, dass der L-Wert 40 oder höher ist, bei Messung mit einem Farbdifferenzmeter.

2. Öl-in-Wasser-emulgiertes Haarkosmetikum nach Anspruch 1, wobei das Mischungsverhältnis der Ölphase einschließlich (a) und (b) 60 - 80 Gew.-% beträgt.

3. Öl-in-Wasser-emulgiertes Haarkosmetikum nach Anspruch 1, wobei ein wasserlösliches Polymer-Verdickungsmittel nicht eingemischt ist oder dessen Mischungsverhältnis 0,5 Gew.-% oder weniger beträgt.

4. Öl-in-Wasser-emulgiertes Haarkosmetikum nach irgendeinem der Ansprüche 1 bis 3, wobei das Hochpolymersilicon von (a) durch die folgende allgemeine Formel (1) wiedergegeben wird, 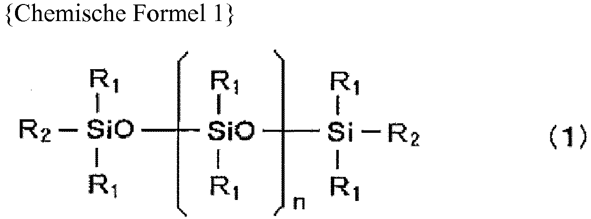 worin R₁ eine Methylgruppe oder teilweise eine Phenylgruppe bedeutet, und R₂ eine Methyl- oder Hydroxylgruppe bedeutet, n eine ganze Zahl von 3.000 bis 20.000 bedeutet.

5. Öl-in-Wasser-emulgiertes Haarkosmetikum nach irgendeinem der Ansprüche 1 bis 3, wobei das aminomodifizierte Silicon und ammoniummodifizierte Silicon von (a) durch die folgende Formel (2) wiedergegeben werden, 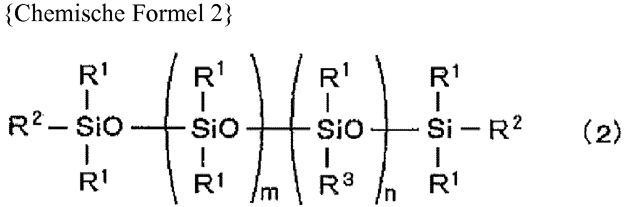 worin R¹ eine Methylgruppe oder teilweise eine Phenylgruppe bedeutet, und R² gleich ist wie R³ oder eine Methyl- oder Hydroxylgruppe bedeutet, R³ einen Substituenten mit einer Aminogruppe oder einer Ammoniumgruppe bedeutet, wiedergegeben durch Formel R⁴Z, worin R⁴ eine zweiwertige Alkylengruppe mit 3 bis 6 Kohlenstoffatomen bedeutet, Z eine einwertige Gruppe bedeutet, gewählt aus einer Gruppe, bestehend aus -NR⁵₂, -N⁺R⁵₃A-, -NR⁵(CH₂)ₐNR⁵₂, -NR⁵(CH₂)ₐN⁺R⁵₃A- und -NR⁵(CH₂)ₐN(R⁵)C=O(R⁶), R⁵ Wasserstoff oder eine Alkylgruppe mit einem Wasserstoff oder 1-4 Kohlenstoffatomen bedeutet, R⁶ eine Alkylgruppe mit 1-4 Kohlenstoffatomen bedeutet, A Cl, Br oder I bedeutet, und a eine ganze Zahl von 2-6 bedeutet, m und n jeweils eine positive ganze Zahl bedeuten, m + n eine ganze Zahl 3.000 - 20.000 bedeutet, und m/n 1/500 - 1/10.000 ist.

6. Öl-in-Wasser-emulgiertes Haarkosmetikum nach irgendeinem der Ansprüche 1 bis 3, wobei das Polyalkylenglykol-modifiziertes Silicon von (a) durch die folgende allgemeine Formel (3) wiedergegeben wird, 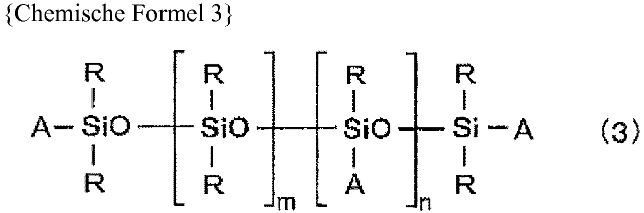 worin R eine Methylgruppe oder Phenylgruppe bedeutet, m eine ganze Zahl von 50 - 1.000 bedeutet, und n eine ganze Zahl von 1-40 bedeutet, A eine Gruppe bedeutet, gewählt aus einer Gruppe, bestehend aus einer Methylgruppe, Phenylgruppe und Polyoxyalkylengruppe, wiedergegeben durch eine allgemeine Formel -C₃H₆O(C₂H₄O)a(C₃H₆O)bR', worin in dieser Formel R' eine Gruppe ist, gewählt aus einer Gruppe, bestehend aus Wasserstoffatom, Acylgruppe und Alkylgruppe mit 1-4 Kohlenstoffatomen, und a und b jeweils eine ganze Zahl 5-50 ist.

7. Öl-in-Wasser-emulgiertes Haarkosmetikum nach irgendeinem der Ansprüche 1 bis 6, wobei das Mischungsverhältnis (Massenverhältnis) zwischen (a) und (b), das heißt (a) : (b) = 1 : 10 - 1:50.

8. Öl-in-Wasser-emulgiertes Haarkosmetikum nach irgendeinem der Ansprüche 1 bis 7, weiterhin umfassend andere Ölkomponenten, verschieden von (a) und (b), gewählt aus Isoparaffinkohlenwasserstoffen mit einem Siedepunkt unter Normaldrucken von 60-260°C, cyclischen Siliconen, wie Octamethylcyclotetrasiloxan, Decamethylcyclopentasiloxan und Dodecamethylcyclohexasiloxan, flüssigen Fetten und Ölen, wie Avocadoöl, Tsubakiöl, Schildkrötenöl, Macadamianussöl, Maisöl, Nerzöl, Olivenöl, Rapsöl, Eigelböl, Sesamöl, Pfirsischkernöl, Weizenkeimöl, Sasanquaöl, Rizinusöl, Leinsamenöl, Sonnenblumenöl, Baumwollsamenöl, Perillaöl, Sojabohnenöl, Erdnussöl, Teesamenöl, Japanischesmuskatnussöl, Reiskleieöl, Chinesisches Gimletöl, Japanisches Gimletöl, Jojobaöl, Keimöl, Triglycerin, Glyceryltrioctanoat und Glyceryltriisopalmitat; festen Fetten und Ölen, wie Kakaobutter, Kokosnussöl, Pferdefett, hydriertes Kokosnussöl, Palmöl, Rindertalg, Hammeltalg, hydrierter Rindertalg, Palmkernöl, Schmalz, Rinderknochenfett, japansiches Kemwachskeimöl, hydriertes Öl, Klauenöl, japanisches Kemwachs und hydriertes Rizinusöl; Wachsen, wie Bienenwachs, Candelillawachs, Baumwollwachs, Carnaubawachs, Myrthenwachs, Baumwachs, Walwachs, Montanwachs, Kleiewachs, Lanolin, Kapokwachs, Lanolinacetat, flüssiges Lanolin, Zuckerrohrwachs, Lanolinfettsäureisopropylester, Hexyllaurat, reduziertes Lanolin, Jojobawachs, Hartlanolin, Shellackwachs, POE-Lanolinalkoholether, POE-Lanolinalkoholacetat, POE-Cholesterolether, Lanolinfettsäurepolyethylenglykol und POE-hydriertes Lanolinalkoholether; Kohlenwasserstoffen, wie flüssiges Petrolatum, Ozocerit, Squalen, Pristan, Parraffin, Ceresin, Squalen, Petrolatum und mikrokristallines Wachs; Fettsäureölen; Alkoholen; und Esterölen, wie Cetyloctanoat und Isopropylmyristat.

9. Öl-in-Wasser-emulgiertes Haarkosmetikum nach irgendeinem der Ansprüche 1 bis 8, wobei der mehrwertige Alkohol (c) eines, zwei oder mehr umfasst, gewählt aus Glycerin, Diglycerin, Propylenglykol, Dipropylenglykol, 1,3-Butylenglykol, Sorbitol, Maltitol, Xylitol, Mannitol und Inositol.

10. Öl-in-Wasser-emulgiertes Haarkosmetikum nach irgendeinem der Ansprüche 1 bis 9, wobei das hydrophile Tensid (d) kationische Tenside, anionische Tenside, ampholytische Tenside und nichtionische Tenside beinhaltet.

11. Öl-in-Wasser-emulgiertes Haarkosmetikum nach Anspruch 10, wobei das hydrophile Tensid (d) einen HLB von 8 oder höher aufweist.

## Revendications

1. Un produit cosmétique pour cheveux émulsionné huile-dans-l'eau transparent ou semi-transparent comprenant de façon caractéristique les suivants (a) - (d) dans lequel le rapport de mélange de la phase huileuse, incluant (a) et (b), est de 50-80% en poids et le rapport de mélange (rapport de masse) entre (a) et (b) est (a) : (b) = 1 : 5 - 1 : 100.
(a) Un, deux ou plusieurs silicones choisies parmi du silicone à haute teneur en polymères, du silicone avec les deux terminaisons modifiés par un groupe hydroxy, du silicone modifié par de l'amino, du silicone modifié par de l'ammonium et du silicone modifié par du polyalkylèneglycol, qui sont semblables à la gomme ou ont une viscosité de 1 million mPa·s (cs) ou plus; 0,1-10% en poids,
(b) diméthylpolysiloxane ayant une viscosité de 100 mPa·s (cs) ou moins; 0,5-79% en poids,
(c) alcool polyhydrique; 1-25% en poids,
(d) tensioactif hydrophile; 1-10% en poids,
dans lequel transparent ou semi-transparent signifie la valeur L est 40 ou plus quand mesuré avec un mètre de différence de couleurs.

2. Le produit cosmétique pour cheveux émulsionné huile-dans-l'eau selon la revendication 1, dans lequel le rapport de mélange de la phase huileuse, comprenant (a) et (b), est de 60 à 80% en poids.

3. Le produit cosmétique pour cheveux émulsionné huile-dans-l'eau selon la revendication 1, dans lequel un épaississant polymère soluble dans l'eau n'est pas mélangé ou son rapport de mélange est de 0,5% en poids ou moins.

4. Le produit cosmétique pour cheveux émulsionné huile-dans-l'eau selon l'une quelconque des revendications 1 à 3, dans lequel le silicone à haute teneur en polymères de (a) est représenté par la suivante formule générale (1), 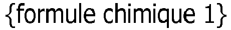 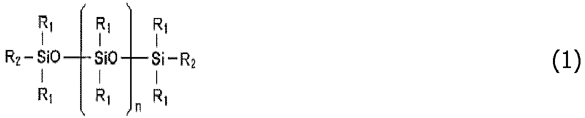 dans laquelle R₁ dénote un groupe méthyle ou partiellement un groupe phényle, et R₂ dénote un groupe méthyle ou un groupe hydroxyle, et n dénote un nombre entier de 3.000 à 20.000.

5. Le produit cosmétique pour cheveux émulsionné huile-dans-l'eau selon l'une quelconque des revendications 1 à 3, dans lequel le silicone modifiée par de l'amino et le silicone modifiée par de l'ammonium de (a) sont représentés par la suivante formule (2), 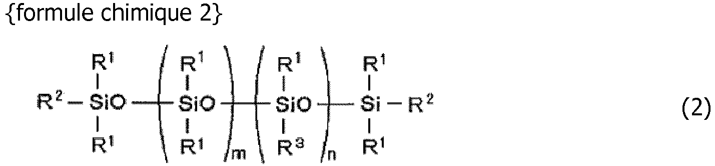 dans laquelle R¹ dénote un groupe méthyle ou partiellement un groupe phényle, et R² est le même que R³ et dénote un groupe méthyle ou un groupe hydroxyle, R³ dénote un substituant ayant un groupe amino ou un groupe ammonium représenté par la formule R⁴Z, dans laquelle R⁴ dénote un groupe alkylène divalent ayant de 3 à 6 atomes de carbone, Z dénote un groupe monovalent choisi parmi le groupe constitué par -NR⁵₂, -N⁺R⁶₃A⁻, -NR⁵(CH₂)ₐNR⁵₂, -NR⁵(CH₂)ₐN⁺R⁶₃A⁻, et -NR⁵(CH₂)ₐN(R⁵)C=O(R⁶), R⁵ dénote un atome d'hydrogène ou un groupe alkyle ayant un atome d'hydrogène ou de 1 à 4 atomes de carbone, R⁶ dénote un groupe alkyle ayant de 1 à 4 atomes de carbone, A dénote du Cl, Br ou I, et a dénote un nombre entier de 2 à 6, m et n dénotent chacun un nombre entier positif, m + n dénotent un nombre entier de 3.000 à 20.000, et n/m est de 1/500 à 1/10.000.

6. Le produit cosmétique pour cheveux émulsionné huile-dans-l'eau selon l'une quelconque des revendications 1 à 3, dans lequel le silicone modifié par du polyalkylèneglycol de (a) est représenté par la suivante formule générale (3), 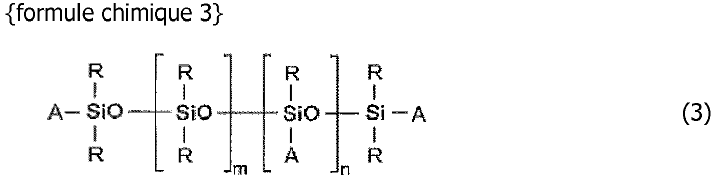 dans laquelle R dénote un groupe méthyle ou un groupe phényle, m dénote un nombre entier de 50 à 1000, et n dénote un nombre entier de 1 à 40, A dénote un groupe choisi parmi le groupe constitué par un groupe méthyle, un groupe phényle, et un groupe polyoxyalkylène représenté par la formule générale -C₃H₆O(C₂H₄O)a(C₃H₆O)bR', dans lequel, dans cette formule, R' représente un groupe choisi parmi le groupe constitué par un atome d'hydrogène, un groupe acyle, et un groupe alkyle ayant de 1 à 4 atomes de carbone, et a et b chacun représentent un nombre entier de 5 à 50.

7. Le produit cosmétique pour cheveux émulsionné huile-dans-l'eau selon l'une quelconque des revendications 1 à 6, dans lequel le rapport de mélange (rapport de masse) entre (a) et (b) est (a) : (b) = 1 : 10 - 1 : 50.

8. Le produit cosmétique pour cheveux émulsionné huile-dans-l'eau selon l'une quelconque des revendications 1 à 7, en outre comprenant d'autres composant d'huile que (a) et (b) choisi parmi les hydrocarbures isoparaffiniques ayant un point d'ébullition sous des pressions normales de 60 à 260 °C, les silicones cycliques telles que l'octaméthylcyclotétrasiloxane, le décaméthylcyclopentasiloxane et le dodécaméthylcyclohexasiloxane, les huiles et huiles liquides comme l'huile d'avocat, l'huile de tsubaki, l'huile de tortue, l'huile de noix de macadamia, l'huile de maïs, l'huile de vison, l'huile de colza, l'huile d'olive, l'huile de jaune d'oeuf, l'huile de sésame, l'huile de noyau d'abricot ou de pêche, l'huile de germe de blé, l'huile de sasanqua, l'huile de ricin, l'huile de lin, l'huile de carthame, l'huile de coton, l'huile de perilla, l'huile de soja, l'huile de cacahuète, l'huile de graines de thé, l'huile de muscade japonaise, l'huile de son de riz, l'huile d'eucalyptus chinois, l'huile d'eucalyptus japonais, l'huile de jojoba, l'huile de germe, la triglycérine, le trioctanoate de glycéryle, et le triisopalmitate de glycéryle; les graisses et les huiles solides telles que le beurre de cacao, l'huile de noix de coco, l'huile de chevaux, l'huile de coco hydrogénée, l'huile de palme, le suif de boeuf, le suif de mouton, le suif de boeuf hydrogéné, l'huile de palmiste, le saindoux, la graisse de boeuf, l'huile de noyau de cire japonaise, l'huile hydrogénée l'huile de de pied de boeuf, la cire de noyau japonais et l'huile de ricin hydrogénée; les cires telles que la cire d'abeille, la cire de candelilla, la cire de coton, la cire de carnauba, la cire de myrique, la cire d'arbre, la cire de baleine, la cire de lignite, la cire de son, la lanoline, la cire de kapok, l'acétate de lanoline, la lanoline liquide, la cire de canne à sucre, l'ester isopropylique d'acide gras de lanoline, le laurate d'hexyle, la lanoline réduite, la cire de jojoba, la lanoline dure, la cire de shellac, l'éther d'alcool de lanoline POE, l'acétate d'alcool de lanoline POE, l'éther de cholestérol POE, le polyéthylène glycol d'acide gras de lanoline, et l'éther d'alcool de lanoline hydrogéné POE; les hydrocarbures tels que l'huile de vaseline, l'ozocérite, le squalène, le pristane, la paraffine, la cérésine, le squalène, le pétrolatum et la cire microcristalline; les huiles d'acides gras; les alcools; et les huiles d'ester telles que l'octanoate de cétyle et le myristate d'isopropyle.

9. Le produit cosmétique pour cheveux émulsionné huile-dans-l'eau selon l'une quelconque des revendications 1 à 8, dans lequel l'alcool polyhydrique (c) comprend un, deux ou plus choisis parmi la glycérine, la diglycérine, le propylèneglycol, le dipropylèneglycol, le 1,3-butylèneglycol, le sorbitol, le maltitol, le xylitol, le mannitol et l'inositol.

10. Le produit cosmétique pour cheveux émulsionné huile-dans-l'eau selon l'une quelconque des revendications 1 à 9, dans lequel le tensioactif hydrophile (d) comprend les tensioactifs cationiques, les tensioactifs anioniques, les tensioactifs ampholytiques et les tensioactifs non ioniques.

11. Le produit cosmétique pour cheveux émulsionné huile-dans-l'eau selon la revendication 10, dans lequel le tensioactif hydrophile (d) a un HLB de 8 ou supérieur.
